# EUROPEAN PATENT SPECIFICATION

(11) **EP 2 586 450 B1**
(45) Date of publication and mention of the grant of the patent: **25.11.2015**
(21) Application number: 10847766.2
(22) Date of filing: 15.12.2010
(51) Int. Cl.: A61K 35/64, A61K 36/185, A61K 36/49, A61K 36/63, A61P 17/02

(54) **CREAM FOR BURNS**
CREME FÜR VERBRENNUNGEN
CRÈME PERMETTANT DE TRAITER LES BRÛLURES

(30) Priority: 17.03.2010 ES 201030393
(43) Date of publication of application: 01.05.2013
(73) Proprietor: Lombardero Calzon, Ricardo, 03010 Alicante (ES); Lombardero Calzon, Daniel, 03010 Alicante (ES)
(72) Inventor: Lombardero Calzon, Ricardo, 03010 Alicante (ES); Lombardero Calzon, Daniel, 03010 Alicante (ES)
(74) Representative: Sempere Massa, Iván Luis
(86) International application number: PCT/ES2010/070832
(87) International publication number: WO 2011/113969

(56) References cited:
- EP-A1- 1 884 245
- ES-A1- 2 052 443
- ES-A1- 2 056 029
- GR-B2- 1 004 069
- US-A- 6 099 866
- US-A1- 2004 101 507
- US-A1- 2005 112 208
- US-A1- 2006 246 030
- US-B1- 6 228 387

## Description

### OBJECT OF THE INVENTION

The present invention refers to a cream for burns, the purpose of which is to immediately relieve the pain and itching of the patient who suffers a burn, effectively and rapidly recovering the surface of burned area without leaving any sign of a scar on the skin.

The invention has application in the pharmaceutical industry, especially in hospitals, serious burns units, chemical and electrical industries, pyrotechnics, fire fighting stations, red cross and the catering and restaurant sector, that is, wherever it can be used for application if a burn might occur.

### BACKGROUND OF THE INVENTION

Currently, the means used for soothing and healing large burns are complex and costly, despite the diversity of the techniques used such as skin grafts, biosynthetic substitutes, cell culture banks, etc., particularly in patients with a high percentage of body burned where plastic and repairing surgery is carried out with the aim of removing irreversibly damaged tissue and definitively covering the injuries, so that repairing plastic surgery is a process that must take place rapidly to minimise the risk of infection, the main cause of death in people suffering from burns.

Obviously, if infection is not prevented, the patient will die from a general infection called septicaemia, which can sometimes be prevented, but on other occasions the patient's suffering is so great that they die from what is called multi-organ failure.

The applicant of this patent does not know of the existence of any current products for healing skin lesions caused by burns that enable rapid recovery of the damaged area and a regeneration of the epidermal tissue, which does not leave any sign of a scar.

### DESCRIPTION OF THE INVENTION

The cream for burns, object of the invention, achieves complete reconstitution of the burned skin, leaving the surface free from any subsequent scar or visible mark of the effect of the burn, preventing and avoiding risk of infection, due to the composition of the ingredients of the cream, allowing this to be considered as a great advance and improvement in the health field.

More specifically, the cream of the invention is obtained from a mixture of virgin olive oil, elderberry bark scrapings, peeled and chopped chestnuts and beeswax in proportions such that the amounts by weight of olive oil is between 18% and 23%, elderberry bark is between 13% and 18%, pealed chestnuts are between 42% and 50% and beeswax is between 15% and 21%, in a total percentage of 100%, so that these components are mixed and held in a container and the mixture is fried to achieve a total and uniform mixture of all the components, achieving a liquid that should be strained or filtered and then cooled so that the cream sets, making it suitable for marketing.

The cream obtained can be kept in the refrigerator for long periods of time without losing its properties, with an estimate that the duration can be up to four years.

The advantages of the cream for burns described above can be summarised as follows:
- Minimal cost of production, given that these are cheap and easily obtained products.
- Simple preparation, given that no more is required than to mix the components and fry them.
- Good use of the cream obtained, given that a small amount can cover a broad area of the affected zone or burn, remaining on it for a long time without needing new applications, as the skin is slow to absorb it, so that after depositing a thin film over the epidermal tissue it does not adhere and repels secretions, providing a nutritional, moisturising and regenerative effect, which soothes the injuries and relieves the patient's pain, thanks to its healing, anti-fungal and anti-bacterial properties.

Nevertheless, in patients with large burns and a high percentage of injured skin, it is necessary to put the cream into a warm water bath, taking it from a solid to a liquid state, so that by bathing and impregnating dressings, gauzes or compresses or by any other means, it can be easily spread over the damaged area, producing the same benefits mentioned above and with a rapid regeneration of the skin and recovery of the patient.

The healing, anti-fungal and anti-bacterial properties of the cream are due to the components used in making it, as the elderberry has bacteriostatic properties, calming and healing, while the chestnuts have astringent and bacteriostatic properties, being effective in the natural treatment of ailments such as cutaneous lesions caused by the effect of the bums.

The preventative and curative properties of virgin olive oil are well known, as is its medicinal use in enemas, some dermatitis and burns, including in cleaning the skin. That is, olive oil has anti-inflammatory and calming properties, serving to calm abrasions, cracks, wounds and soothing eczemas and other skin diseases.

Finally, beeswax is used in cleaning of the epidermis, especially feeding the dermis and preventing cutaneous aging, also having analgesic, anti-inflammatory and healing properties. It should also be pointed out that beeswax increases blood circulation in the area where it is applied, thereby facilitating transport of oxygen and nutrients, in addition to helping in the dissolution of fats, also having antibiotic properties and a high regenerative power, increasing healing in this way.

Finally, the cream for burns of the invention is a creamy product, yellow in colour, constituting a product for accelerated healing of all types of burns and having an instant calming action, with excellent results both for the effect of cold and of heat and sunburn, abrasive and chemical products, being equally applicable and with great results in scratches, bumps, bruises, strains, sprains and haemorrhoids, given its high anti-inflammatory properties.

### EXAMPLES OF PRACTICAL EMBODIMENT

### Example 1.

The cream for burns of the invention, containing 20.52% virgin olive oil, 15.36% elderberry bark, 46.17% peeled chestnuts and 17.95% beeswax by weight in a total percentage of 100%, so that these components in the cited proportions were mixed together, where the chestnuts were previously peeled and chopped, and the elderberry bark supplied as scrapings, and the mixture placed in a container and fried for an appropriate time to achieve a homogenous mixture giving a liquid as a result, that was subsequently strained or filtered to achieve definitive homogenisation of all the substances or components in it, and finally the product was put into a container for marketing, after first cooling it, and it was possible to keep the product in the refrigerator for long periods of time.

### Example 2.

A quarter of a litre of virgin olive oil, or 200 g by weight, 150 g of elderberry bark scrapings, 450 g or previously peeled and chopped chestnuts and 175 g of beeswax were mixed, then fried until obtaining a golden colour, the liquid component was filtered and/or strained and subsequently left to cool and thereby the cream or product obtained. This resulted in accelerating the healing of all types of burns and an instant calming action.

## Claims

1. Cream for burns, constituting a product applicable in healing all types of burns and in an instant calming action, being equally applicable to scratches, bumps, bruises and sprains, is **characterised in that** it is constituted by a mixture of virgin olive oil, elderberry bark scrapings, previously peeled and chopped chestnuts and beeswax, in a percentage by weight of between 18% and 23% of virgin olive oil; between 13% and 18% of elderberry bark scrapings; between 42% and 50% of peeled chestnuts; and between 15% and 21% of bees wax, **characterized by** the process that this mixture is fried to achieve a liquid and is subsequently strained and/or filtered, before its definitive cooling, during which the liquid congeals to form the cream.

## Patentansprüche

1. Creme für Verbrennungen, welche ein Produkt darstellt, das für die Heilung aller Art von Verbrennungen und für eine sofortige beruhigende Wirkung anwendbar ist, wobei sie ebenso für Kratzwunden, Beulen, Hämatome und Verstauchungen anwendbar ist, **dadurch gekennzeichnet, dass** sie aus einer Mischung aus nativem Olivenöl, Abrieben von Holunderrinden, zuvor abgeschälten und zerkleinerten Kastanien und Bienenwachs, in einem Gewichtsanteil von zwischen 18% und 23% von nativem Olivenöl; zwischen 13% und 18% von Abrieben von Holunderrinden; zwischen 42% und 50% von abgeschälten Kastanien; und zwischen 15% und 21% von Bienenwachs besteht, **gekennzeichnet durch** das Verfahren, in welchem diese Mischung gebraten wird, um eine Flüssigkeit zu erreichen und später gesiebt und/oder filtriert wird, bevor sie definitiv abgekühlt wird, wobei während der Abkühlung die Flüssigkeit fest wird, um die Creme zu bilden.

## Revendications

1. Crème pour brûlures, constituant un produit applicable à la cicatrisation de tous types de brûlures et à une action calmante instantanée, étant également applicable à des égratignures, des bosses, des hématomes et des entorses, est **caractérisée en ce qu'**elle est constituée d'un mélange d'huile d'olive vierge, d'écorce de sureau râpée, de châtaignes préalablement pelées et hachées et de la cire d'abeille, dans un pourcentage en poids d'entre 18% et 23% d'huile d'olive vierge; entre 13% et 18% d'écorce de sureau râpée; entre 42% et 50% de châtaignes pelées; et entre 15% et 21% de cire d'abeille, **caractérisée par** le procédé dans lequel ce mélange est frit pour obtenir un liquide et par la suite est tamisé et/ou filtré, avant son refroidissement définitif, pendant lequel le liquide se solidifie pour former la crème.
